(19) Europäisches Patentamt
European Patent Office
Office européen des brevets

(11) Veröffentlichungsnummer : **0 151 986**
**B1**

(12) EUROPÄISCHE PATENTSCHRIFT

(45) Veröffentlichungstag der Patentschrift :
05.04.89

(21) Anmeldenummer : 85100745.0

(22) Anmeldetag : 25.01.85

(51) Int. Cl.⁴ : **B 01 J 23/78**, **B 01 J 23/84**,
**B 01 J 27/185**, **B 01 J 23/70**,
**B 01 J 23/16**, **C 07 C 85/12**,
**C 07 C 87/14**

## ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | Seite | Spalte | Zeile | LAUTET BERICHTIGT:<br>SHOULD READ :<br>DEVRAIT ETRE LU : |
|---|---|---|---|---|
| balt Alkali- und | 2 | 1 | 26 | balt, Alkali- und |
| von größer $294.10^2$ KPa | 2 | 2 | 40 | von größer $294.10^2$ kPa |
| KPa ( 350 bis 1 500 kp/cm$^2$). | 2 | 2 | 42 | kPa (350 bis 1 500 kp/cm$^2$). |
| $\geqslant 294.10^2$ KPa | 3 | 4 | 38 | $\geqslant 294.10^2$ kPa |
| $784.10^2$ bis $1\ 274.10^2$ KPA | 4 | 5 | 1 | $784.10^2$ bis $1\ 274.10^2$ kPa |
| $< 245.10^2$ KPa (300 | 4 | 5 | 13 | $< 245.10^2$ kPa (300 |
| $Co(NO_3)_2$ x $6H_2O$ 261 g | 4 | 6 | 44 | $Co(NO_3)_2$ x $6H_2O$, 261 g |
| beträgt $294.10^2$ KPa | 5 | 7 | 15 | beträgt $294.10^2$ kPa |
| von $607,6.10^2$ KPA (620 | 5 | 7 | 25 | von $607,6.10^2$ kPa (620 |
| Daraufhin wird geformte | 5 | 7 | 31 | Daraufhin wird die geformte |
| Druckhärte von $931.10^2$ KPa | 5 | 7 | 35 | Druckhärte von $931.10^2$ kPa |
| Druckhärte von $1\ 117.10^2$ KPa | 5 | 7 | 38 | Druckhärte von $1\ 117.10^2$ kPa |
| wird auf $294.10^2$ KPa | 5 | 8 | 6 | wird auf $294.10^2$ kPa |
| mehr als $294.10^2$ KPa | 5 | 8 | 54 | mehr als $294.10^2$ kPa |
| $343.10^2$ bis $1\ 470.10^2$ KPa | 5 | 8 | 58 | $343.10^2$ bis $1\ 470.10^2$ kPa |
| $588.10^2$ bis $784.10^2$ KPa | 3 | 4 | 51 | $588.10^2$ bis $784.10^2$ kPa |
| auf Werte $\leqslant 24,5.10^2$ KPa | 5 | 8 | 12 | auf Werte $\leqslant 24,5.10^2$ kPa |

Tag der Entscheidung
über die Berichtigung
Date of decision on
rectification:
) 27.06.89

Ausgabe- und Veröffentlichungstag:
Issue and publication ) 23.08.89
date:
Date d'édition et de

Patbl.Nr.)
EPB no:) 89/34
Bull. no:)

Europäisches Patentamt

European Patent Office

Office européen des brevets

(19)

(11) Veröffentlichungsnummer : **0 151 986**
**B1**

(12) **EUROPÄISCHE PATENTSCHRIFT**

(45) Veröffentlichungstag der Patentschrift :
05.04.89

(21) Anmeldenummer : 85100745.0

(22) Anmeldetag : 25.01.85

(51) Int. Cl.⁴ : **B 01 J 23/78**, B 01 J 23/84,
B 01 J 27/185, B 01 J 23/70,
B 01 J 23/16, C 07 C 85/12,
C 07 C 87/14

(54) Geformte Katalysatormassen, deren Herstellung und Verwendung.

(30) Priorität : 01.02.84 DE 3403377

(43) Veröffentlichungstag der Anmeldung :
21.08.85 Patentblatt 85/34

(45) Bekanntmachung des Hinweises auf die Patenterteilung : 05.04.89 Patentblatt 89/14

(84) Benannte Vertragsstaaten :
BE CH DE FR GB IT LI NL

(56) Entgegenhaltungen :
DE—A— 2 654 028
DE—A— 2 952 061
DE—B— 1 266 736
DE—B— 2 021 522
US—A— 4 140 720

(73) Patentinhaber : BASF Aktiengesellschaft
Carl-Bosch-Strasse 38
D-6700 Ludwigshafen (DE)

(72) Erfinder : Frank, Gerhard, Dr.
Heidelberger Strasse 11
D-6945 Hirschberg (DE)
Erfinder : Neubauer, Gerald, Dr.
Mozartstrasse 24
D-6940 Weinheim (DE)
Erfinder : Duffner, Paul, Dr.
Bozener Strasse 14
D-6700 Ludwigshafen (DE)
Erfinder : Wilfinger, Hans Joerg, Dr.
Buschstrasse 11
D-6707 Schifferstadt (DE)

## Beschreibung

Gegenstand der Erfindung sind geformte Katalysatormassen enthaltend metallische Kobalt- und/oder Nickelteilchen und Gleitmittel sowie deren Herstellung und Verwendung.

Bei der Herstellung von Aminen durch Hydrieren von Nitrilen, z. B. Hexamethylendiamin aus Adiponitril werden Kobalt enthaltende Katalysatoren wegen ihrer hohen Selektivität bevorzugt verwendet. Solche Verfahren sind beispielsweise aus den DE-Cen 1 072 972 und 1 259 899 bekannt. Die Lebensdauer der verwendeten Kobaltkatalysatoren entspricht jedoch nicht mehr den technischen Anforderungen. Die verwendeten Katalysatoren haben den Nachteil, daß sie im Laufe ihres Gebrauchs zerfallen, wodurch ihre Wirkungsweise beeinträchtigt wird. Aus der FR-A 1 407 414 ist auch schon bekannt, daß man Kobaltkatalysatoren, die durch Verpressen von Kobaltoxid mit Graphit hergestellt worden sind, für die Hydrierung von Adipodinitril zu Hexamethylendiamin verwendet. Solche Katalysatoren verlieren beim Gebrauch rasch ihre Härte und zerfallen, wodurch ihre Dauergebrauchsfähigkeit sehr beschränkt ist. Aus der DE-A 26 54 028 sind auch bereits geformte Katalysatormassen, die metallisches Kobalt Alkali- und Erdalkalioxide sowie Graphit enthalten, bekannt. Diese Katalysatoren führen bei der Hydrierung von Adipodinitril jedoch zur vermehrten Bildung von cyclischen Produkten.

Es war die technische Aufgabe gestellt, geformte Kobalt und/oder Nickel enthaltende Katalysatormassen zur Verfügung zu stellen, die sich durch hohe Aktivität und lange Lebensdauer insbesondere hohe Druckhärte, mechanische Beständigkeit und Abriebfestigkeit auszeichnen.

Diese Aufgabe wird gelöst durch geformte Katalysatormassen, enthaltend metallische Kobalt- und/oder Nickelteilchen und ein Gleitmittel, dadurch gekennzeichnet, daß die metallischen Kobalt- und/oder Nickelteilchen durch Reduktion von Kobalt- und/oder Nicheloxidteilchen mit einem Gehalt an Alkali- und/oder Erdalkalioxiden von weniger als 0,1 Gew.% bei einer Temperatur von ≤ 500 °C erhalten worden sind, und die geformten Katalysatormassen eine Druckhärte von mehr als 294.10² KPa (300 kp/cm²) haben.

Gegenstand der Erfindung ist ferner ein Verfahren zur Herstellung von geformten Katalysatormassen durch Reduktion von Kobalt und/oder Nickel-oxid, dadurch gekennzeichnet, daß man

a) Kobalt und/oder Nickeloxidteilchen durch Behandeln mit Wasserstoff bei einer Temperatur von 250 bis 500 °C zu metallischen Kobalt- und/oder Nickelteilchen reduziert

b) die metallischen Kobalt- und/oder Nickelteilchen durch Behandeln mit molekularem Sauerstoff enthaltenden Inertgas passiviert

c) die passivierten Kobalt- und/oder Nickelteilchen mit Gleitmitteln zu Formkörpern verpreßt und

d) die passivierte Kobalt- und/oder Nickelteilchen sowie Gleitmittel enthaltenden Formkörper durch Behandeln mit Wasserstoff bei einer Temperatur von 250 bis 500 °C aktiviert.

Ferner ist ein Gegenstand der Erfindung die Verwendung der metallische Kobalt- und/oder Nickelteilchen enthaltenden geformten Katalysatormassen zur Hydrierung von organischen Verbindungen.

Die neuen metallische Kobalt und/oder Nickelteilchen enthaltenden geformten Katalysatormassen mit hoher Druckhärte haben eine lange Lebensdauer und zeichnen sich auch nach längerem Gebrauch durch überlegene mechanische Eigenschaften aus. Die neuen Katalysatoren haben ferner den Vorteil, daß sie nicht zerfallen, wenig Abrieb haben und auch nach langem Gebrauch noch eine hohe Selektivität haben.

Die erfindungsgemäßen Katalysatormassen enthalten metallische Kobalt- und/oder Nickelteilchen, die aus Kobalt- und/oder Nickeloxidteilchen durch Kontakt mit Wasserstoff bei einer Temperatur von ≤ 500 °C erhalten worden sind. Vorteilhaft weisen die metallischen Kobalt- und/oder Nickelteilchen einen Reduktionsgrad von größer 80 %, insbesondere größer 95 % auf. Unter Reduktionsgrad ist der Anteil des verfügbaren Kobalts und/oder Nickels in Prozent zu verstehen, der in metallischer Form vorliegt. Vorteilhaft beträgt der Gehalt an Alkali- und/oder Erdalkalioxiden weniger als 0,1 Gew.%.

Die erfindungsgemäßen Katalysatormassen enthalten ferner Gleitmittel, z. B. anorganische Stoffe mit Gitterstruktur, wie Talkum oder Graphit. Vorteilhaft enthalten die Katalysatoren 1 bis 5 Gew.% an Gleitmittel, bezogen auf die gesamte Katalysatormasse, aus aktiver Masse enthaltend metallische Kobalt- und/oder Nickelteilchen sowie Gleitmittel. Besonders bewährt hat sich Graphit als Gleitmittel.

Die erfindungsgemäßen Katalysatormassen haben eine Druckhärte von größer 294.10² KPa (300 kp/cm²), insbesondere von 343.10² bis 1 470.10² KPa (350 bis 1 500 kp/cm²).

Bevorzugt werden Kobalt- und/oder Nickeloxidteilchen mit einer Teilchengröße von 0,1 bis 5 insbesondere 0,2 bis 2 verwendet. Die als Ausgangsstoffe verwendeten Kobalt- und/oder Nickeloxidteilchen können weitere aktivierende Zusätze, wie Mangan, Chrom und/oder Kupferoxid sowie Pyro- und/oder Polyphosphorsäure enthalten. Bevorzugt enthalten die Oxidteilchen 80 bis 99 Gew.% Nickel und/oder Kobalt in Form ihrer Oxide berechnet als Metall, 0,5 bis 10 Gew.% Mangan, Chrom und/oder Kupfer in Form ihrer Oxide, berechnet als Metall sowie 0,5 bis 10 Gew.% Poly- und/oder Pyrophosphorsäure. Es versteht sich, daß die daraus resultierende katalytisch aktive Masse abzüglich Gleitmittel die entsprechende Zusammensetzung in fertigen Katalysator hat. Es ist davon auszugehen, daß im fertigen Katalysator Kupfer als Metall vorliegt, während nicht bekannt ist, in welcher Form Mangan und/oder Chrom im fertigen Katalysator vorliegen.

Die fertigen Katalysatoren bestehen somit im wesentlichen aus metallischen Kobalt- und/oder Nickelteilchen, geringen Mengen Kobalt und/oder Nickeloxid entsprechend dem Reduktionsgrad, gegebenenfalls den vorgenannten Zusätzen und einem Gleitmittel.

Die erfindungsgemäßen Katalysatormassen sind geformt, z. B. als Kugeln, Tabletten oder Stränge.

Bei der Herstellung der Oxidteilchen geht man in der Regel von wäßrigen Lösungen von Kobalt und/oder Nickelsalzen aus. Falls aktivierende Zusätze, wie Mangan, Chrom und/oder Kupfer mitverwendet werden sollen, benutzt man zusätzlich wäßrige Lösungen von Salzen dieser Metalle. Geeignete Salze sind beispielsweise Nitrate, Sulfate, Chloride oder Salze mit niederen Fettsäuren. Soll der Katalysator Pyro- oder Polyphosphorsäure enthalten, so wird Phosphorsäure mitverwendet, die bei der weiteren Verarbeitung in Pyro- oder Polyphosphosäure übergeht. Die Ausgangslösungen werden zweckmäßig zunächst im sauren Bereich, etwa im pH-Bereich von 1 bis 2 vereinigt. Dann fällt man in der Regel mittels wäßriger Alkalilauge, insbesondere 10 bis 25 gew.%iger, oder wäßriger Alkalicarbonatlauge, insbesondere 5 bis 25 gew.%iger, ein Gemisch der Metalloxide, Hydroxide und Carbonate zusammen mit den Säurebestandteilen aus. Es ist auch möglich, die wäßrigen Lösungen der Metallverbindungen in die alkalische Fällösung einlaufen zu lassen. Es hat sich als vorteilhaft erwiesen, die Mengenverhältnisse von sauer Lösung und alkalischem Fällmittel so zu wählen, daß sich im Reaktionsgemisch am Ende ein pH-Wert von 7,0 bis 7,5 einstellt. Das ausgefallene Oxid, Hydroxid und/oder Carbonatgemisch wird abfiltriert, mit Wasser von Fremdsalzen freigewaschen und getrocknet. Anschließend wird das Gemisch durch Erhitzen in die entsprechenden Oxide übergeführt. Im allgemeinen sind hierfür Temperaturen von 250 bis 500 °C ausreichend. So erhaltene Masse wird dann z. B. auf die geeignete Teilchengröße, wie vorgenannt, gemahlen. Nach einer vorteilhaften Arbeitsweise wird die oxidische Masse nach Anteigen, z. B. mit Wasser, zu Strängen verformt und die so erhaltenen Formkörper noch einmal bei einer Temperatur von 300 bis 800 °C getempert und anschließend auf die angegebene Teilchengröße gemahlen. Die so erhaltenen Kobalt- und/oder Nickeloxid enthaltenden Teilchen haben einen Gehalt an Alkali- und/oder Erdalkalioxiden von weniger als 0,1 Gew.%.

Die Kobalt- und/oder Nickeloxid enthaltenden Teilchen werden mittels Wasserstoff, z. B. in der Wirbelschicht, im Drehrohrofen oder vorzugsweise in einem gerührten Festbett bei einer Temperatur von 250 bis 500 °C, insbesondere 300 bis 450 °C z. B. innerhalb von 3 bis 36 Stunden reduziert. Vorteilhaft wendet man einen trockenen Wasserstoffstrom an, der frei von wesentlichen Mengen an Wasser ist, wobei man eine relativ große Wasserstoffströmungsgeschwindigkeit einhält. Es hat sich bewährt, wenn man mindestens einen 60-fachen Wasserstoffüberschuß anwendet. Vorteilhaft reduziert man so lange, bis ein Reduktionsgrad von größer gleich 80 %, insbesondere größer gleich 95 % erreicht ist.

Anschließend werden die Metallteilchen durch Passivierung stabilisiert. Hierunter versteht man das Umhüllen der Metallteilchen mit einer Oxidschicht durch kontrollierte Oxidation um die durch die große freie Oberfläche der kleinen Teilchen bedingte Pyrophorität zu beseitigen. Dies wird durch Behandeln mit einem molekularen Sauerstoff enthaltenden Inertgas erreicht. Vorteilhaft verwendet man hierzu 0,1 bis 1,0 Vol.% molekularen Sauerstoff enthaltende Inertgase. Inertgase sind beispielsweise Stickstoff oder Edelgase. Besonders bewährt hat es sich, wenn man ein Luft/Stickstoffgemisch verwendet. Die Passivierung wird z. B. erreicht durch Überleiten eines Luft/Stickstoffgemisches unter genauer Einhaltung einer vorzugsweise 100 °C insbesondere 80 °C nicht übersteigenden Temperatur über das Metallpulver. Nach der Stabilisierung soll der Reduktionsgrad nicht unter 80 %, vorzugsweise nicht unter 90 % liegen. Die stabilisierten Kobalt- und/oder Nickelteilchen haben einen Durchmesser von 0,05 bis 2 μm, insbesondere von 0,2 bis 1,2 μm.

Die so passivierten metallischen Kobalt- und/oder Nickelteilchen werden mit einem inerten Gleitmittel, vorzugsweise Graphit vermischt. Hierbei wendet man vorteilhaft 1 bis 5 Gew.% Gleitmittel, bezogen auf die Summe aus Metallteilchen enthaltenden Masse und Gleitmittel an. Das Gemisch aus passivierten Kobalt- und/oder Nickelteilchen und Gleitmittel wird vorteilhaft unter Stickstoffabdeckung zu Formkörpern verarbeitet, z. B. zu Tabletten verpreßt. Die Druckhärte der Formkörper ist zweckmäßig ≥ 294.10² KPa (300 kp/cm²).

Die so erhaltenen Formkörper werden durch Behandeln mit Wasserstoff in relativ großem Überschuß, z. B. dem 60-fachen Überschuß in Abwesenheit von wesentlichen Mengen Wasser bei einer Temperatur ≤ 500 °C, z. B. 250 bis 500 °C, vorzugsweise 300 bis 360 °C bei Atmosphärendruck oder unter erhöhtem Druck, z. B. 100 bis 150 bar aktiviert. Hierbei soll ein Reduktionsgrad von vorteilhaft größer 95 % erreicht werden. Durch die Aktivierung steigt die Druckhärte der Formkörper, z. B. von 294.10² auf 588.10² bis 784.10² KPa (300 auf 600 bis 800 kp/cm²).

Es hat sich besonders bewährt, wenn man die fertigen aktivierten geformten Katalysatormassen mindestens noch einmal durch Behandeln mit molekularem Sauerstoff enthaltendem Inertgas, wie vorgehend beschrieben, passiviert, wobei ein Reduktionsgrad von 80 % nicht unterschritten werden soll und anschließend wiederum durch Behandeln mit Wasserstoff bei einer Temperatur ≤ 500 °C, vorzugsweise 300 bis 360 °C, wie vorgehend beschrieben, aktiviert. Dieser Vorgang wird zweckmäßig 1- bis 5-mal, insbesondere 2- bis 4-mal wiederholt. Hierbei steigt die Druckhärte mit jedem Zyklus an und erreicht Werte von

784.10$^2$ bis 1 274.10$^2$ KPa (800 bis 1 300 kp/cm$^2$).

Die erfindungsgemäßen Katalysatormassen zeichnen sich durch eine hohe mechanische Stabilität aus, die dadurch erreicht wird, daß man die Verformung der Formkörper zusammen mit Gleitmitteln nicht auf der Stufe der Oxide vornimmt, sondern erst nach deren Reduktion zu Kobalt- und/oder Nickelteilchen und nachfolgender Passivierung. Geht man von Formlingen aus, die aus Kobalt- und/oder Nickeloxidteilchen und Gleitmitteln hergestellt und anschließend aktiviert worden sind, so geht die Druckhärte der Formlinge z. B. von 294.10$^2$ auf < 245.10$^2$ KPa (300 kp/cm$^2$ auf < 25 kp/cm$^2$) nach Erreichen eines Reduktionsgrades von 95 % zurück. Die Lebensdauer solcher Katalysatoren wird hiedurch erheblich erniedrigt.

Die erfindungsgemäßen Katalysatormassen eigenen sich vorteilhaft zur Hydrierung von organischen Verbindungen, insbesondere von olefinischen Kohlenstoffdoppel- oder Kohlenstoffdreifachbindungen, Aldehyd-, Keto-, oder Carbonsäureestergruppen zu den entsprechenden Alkoholen oder Nitrilgruppen zu den entsprechenden Aminen. Ferner für die aminierende Hydrierung von Alkoholen, Aldehyden oder Ketonen in Gegenwart von Ammoniak zu den entsprechenden Aminen.

Der erfindungsgemäße Katalysator ist vorteilhaft geeignet für die Herstellung von Aminen durch Hydrierung von aliphatischen, cycloaliphatischen, araliphatischen oder aromatischen Nitrilen mit bis zu 20 Kohlenstoffatomen. Die Nitrilgruppe kann ein oder mehrmals im Molekül vorkommen. Für die Hydrierung eignen sich gesättigte oder olefinisch ungesättigte Nitrile. Sie können auch unter Reaktionsbedingungen inerte Substituenten haben, wie über Etherbrücken gebundene Alkylreste mit 1 bis 4 Kohlenstoffatomen. Besonders bevorzugte Ausgangsstoffe sind Alkannitrile oder Alkandinitrile mit 3 bis 18 Kohlenstoffatomen. Geeignete Nitrile sind beispielsweise Propionitril, Acetonitril, Acrylnitril, Benzylcyanid, Benzonitril, Glutarsäuredinitril, But-2-endinitril(1,4) oder Adipinsäuredinitril. Besondere technische Bedeutung hat das Verfahren bei der Hydrierung von Adipinsäuredinitril zu Hexamethylendiamin erlangt. Die Umsetzung führt man im allgemeinen unter Drücken von 150 bis 400 bar, vorzugsweise 200 bis 300 bar durch und hält vorzugsweise Temperaturen von 80 bis 140 °C, insbesondere von 100 bis 120 °C ein. Die Umsetzung wird in Gegenwart von Ammoniak durchgeführt. Es hat sich bewährt, wenn das Volumenverhältnis von Nitril zu Ammoniak von 1 : 2 bis 1 : 20, vorzugsweise 1 : 6 bis 1 : 12 beträgt. Es ist auch möglich Ammoniak teilweise durch zurückgeführtes rohes Hydriergemisch, das im wesentlichen aus Amin und Ammoniak besteht, zu ersetzen.

Die erfindungsgemäßen geformten Katalysatormassen eigenen sich ferner zur Herstellung von Alkoholen durch Hydrierung von Aldehyden, Ketonen oder Carbonsäureestern. Als Ausgangsstoffe verwendet man bevorzugt aliphatische, cycloaliphatische, araliphatische oder aromatische Aldehyde oder Ketone sowie Carbonsäureester mit ($C_1$- bis $C_4$-Alkanolen) mit bis zu 20 Kohlenstoffatomen. Die Carbonylgruppe oder Carbonsäureestergruppe kann ein- oder mehrmals im Molekül vorkommen. Für die Hydrierung eignen sich gesättigte oder olefinisch ungesättigte Aldehyde, Ketone oder Carbonsäureester. Sie können auch unter Reaktionsbedingungen inerte Substituenten haben, wie über Etherbrücken gebundende Alkylreste mit 1 bis 4 Kohlenstoffatomen oder aromatisch gebundene Halogenatome. Geeignete Aldehyde sind beispielsweise Acetaldehyd, Propionaldehyd, Hexanal, Octanal, Decanal, 2-Ethylhexen-2-al-1, Benzaldehyd, 2-Phenylacetaldehyd, Parachlorbenzaldehyd, Acetophenon, Methyldecylketon, Isobutyraldehyd, 2-Ethylhexanal, 2-Ethyl-4-methyl-penten-2-al-1, ferner Oxoreaktionsgemische, die neben Butyraldehyden bei der Oxoreaktion anfallende Nebenprodukte wie Acetale, Ester und Ether enthalten. Typische Gemische enthalten beispielsweise 4 bis 80 Gew.% Butyraldehyde, 10 bis 55 Gew.% Butanole, 3 bis 10 Gew.% $C_1$- bis $C_4$-Carbonsäureester, 0,1 bis 2 Gew.% Acetale, 0,2 bis 1 Gew.% Ether und Ketone. Ferner sind geeignet Aldehydgemische, wie sie bei der Hydroformylierung von Olefinschnitten mit $C_8$- bis $C_{10}$-Olefinen oder $C_{10}$- bis $C_{12}$-Olefinen anfallen. Besondere technische Bedeutung ist die Hydrierung von 2-Ethylhexen-2-al-1 oder Aldehyden, die unmittelbar durch Oxoreaktion von Olefinen mit Kohlenmonoxid und Wasserstoff hergestellt werden. Ferner für die Hydrierung von Alkandicarbonsäureestergemischen zu den entsprechenden Alkandiolen. Die Hydrierung führt man im allgemeinen bei Drücken von 30 bis 300 bar, insbesondere 30 bis 300 bar durch. Zweckmäßig hält man Temperaturen von 80 bis 200 °C, insbesondere 100 bis 180 °C ein.

Der Gegenstand der Erfindung sei an folgenden Beispielen veranschaulicht.

Beispiel 1

4 480 g Kobaltnitrat Co(NO$_3$)$_2$ × 6 H$_2$O 261 g Manganitrat Mn (NO$_3$)$_2$ · 6 H$_2$O und 47 g Phosphorsäure 85 gew.%ig werden in 10 l Wasser gelöst. Diese Lösung läßt man in eine Lösung von 1 900 g Natriumcarbonat Na$_2$CO$_3$ in 10 l Wasser langsam unter Rühren einlaufen. Nach beendetem Zulauf der Metallsalzlösung ist der pH des Gemisches auf 7,0 gefallen. Nach dem Erkalten wird der Niederschlag durch Absaugen abgetrennt und solange mit Wasser gewaschen, bis der Niederschlag frei von Natriumionen ist. Der Filterkuchen wird getrocknet und anschließend auf 300 °C erhitzt, bis die Masse frei von Carbonat ist. Das Oxidgemisch wird dann mit soviel Wasser angeteigt, daß eine knetbare Masse entsteht. Diese wird in einer Presse zu Strängen verformt. Die Stränge werden dann bei 450 °C im Muffelofen für 24 Stunden getempert. Die erhaltenen Stränge werden zu Teilchen einer Größe von 0,1 bis 1,2 µm gemahlen.

600 kg der so hergestellten Kobaltoxid enthaltenen Teilchen werden in einem gerührten Festbett bei 400 °C 38 Stunden mit 400 Nm³/h Wasserstoff zu metallischem Kobalt mit einem Reduktionsgrad bezogen auf Kobalt größer 95 % reduziert (stöchiometrischer Wasserstoffüberschuß 64). Anschließend wird in einem Stickstoff-Luft-Gemischt das pyrophore Metallpigment bei einer Temperatur von 60 °C mit einer stabilisierenden Oxidschicht überzogen und passiviert, wobei der Reduktionsgrad nicht unter 90 % absinken soll.

Zur Herstellung von geformten Massen mit einem Durchmesser von 5 mm und einer Höhe von 4 mm wird das passivierte pulverförmige Metallpigment mit 2 Gew.% Graphit vermischt und unter Stickstoffabdeckung tablettiert. Die Druckhärte der Tabletten beträgt 294.10² KPa (300 kp/cm²).

In einem Hochdruckgefäß werden 350 l der so hergestellten Formkörper bei 360 °C unter einem Druck von 150 bar mit hohem Wasserüberschuß für 24 Stunden zum Zweck der Aktivierung behandelt. Der Wasserstoff wird dabei im Kreis über einen Kondensator zur Abscheidung des Reduktionswassers geführt. Die so erhaltene geformte Katalysatormasse hat einen Reduktionsgrad von 98 % und eine Druckhärte von 607,6.10² KPa (620 kp/cm²). Anschließend wird die geformte Katalysatormasse durch Behandeln mit einem Gemisch aus Stickstoff und Luft mit einem Sauerstoffgehalt von 0,5 Vol.% bei einer Temperatur von 60 °C passiviert, wobei der Reduktionsgrad 90 % nicht unterschreitet. Daraufhin wird geformte Katalysatormasse wiederum bei 360 °C und unter einem Druck von 150 bar wie oben beschrieben mit Wasserstoff aktiviert. Die so erhaltenen Formlinge haben eine Druckhärte von 931.10² KPa (950 kp/cm²). Nach der zweiten Wiederholung des Zyklus Passivieren Aktivieren haben die Formkörper eine Druckhärte von 1 117.10² KPa (1 140 kp/cm²).

Nach dem Abkühlen des Katalysators wird das Reaktionsgefäß in Rieselfahrweise bei 270 bar Wasserstoffdruck stündlich mit einem Gemisch von 85 l Adipinsäuredinitril und 255 l flüssigem Ammoniak und 850 l rohem Hydriergemisch unter Kreisgasfahrweise des Wasserstoffs (400 Nm³/h) beschickt. Die Temperatur des Zulaufgemischs beträgt 65 °C und die des Reaktorausgangs liegt bei 95 °C. Es entstehen hot spot-Temperaturen von maximal 100 °C. Die gaschromatographische Analyse des rohen Hexamethylendiamins nach Verdampfen des Ammoniaks aus dem Hydriergemisch ergibt 0,02 Gew.% Hexylamin, 0,05 Gew.% Azacycloheptan, 0,06 Gew.% 1,2-Diaminocyclohexan, 0,003 Gew.% 2-Aminomethylcyclopentylamin und 99,36 Gew.% Hexamethylendiamin sowie einen Aminocapronitrilgehalt von < 0,01 Gew.%. Der vorwiegend aus Bishexamethylentriamin bestehende Destillatinsrückstand liegt bei 0,3 Gew.%. Es errechnet sich hieraus eine Selektivität von 99,5 % Hexamethylendiamin. Der Katalysator hatte nach einer Laufzeit von 520 Tagen eine unveränderte Aktivität und Selektivität ohne jegliche Regenerierung. Die geformte Katalysatormasse hatte nach dieser Laufzeit eine Druckhärte von > 882.10² KPa (900 kp/cm²).

Vergleichsbeispiel 1

Das nach Beispiel 1 hergestellte Kobaltoxid wird nach Zusatz von 2 % Graphit zu Tabletten (Durchmesser 5 mm, Höhe 4 mm) verpreßt. Die Druckhärte wird auf 294.10² KPa (300 kp/cm²) eingestellt. Danach werden die Tabletten mit Wasserstoff bei 360 °C auf einen Reduktionsgrad von ≥ 95 % gebracht. Durch die Reduktion des Kobaltoxids sinkt die Druckhärte der Tabletten drastisch auf Werte ≤ 24,5.10² KPa (25 kp/cm²). Die Tabletten zerfallen bei Berührung der zylindrischen Mantelfläche leicht in Scheibchen.

Im Unterschied zu Tabletten, die aus bereits zu Kobalt reduziertem Kobaltoxid hergestellt wurden, kann die mechanische Stabilität dieser Tabletten durch wiederholtes Passivieren und Reduzieren nicht verbessert werden.

Vergleichsbeispiel 2

Ein Hochdruckgefäß von 2 m Länge und 42 mm Durchmesser wird mit 3 l eines nach Beispiel 1 der DE-A-26 54 028 hergestellten passivierten Kobaltkatalysators beschickt. Der Katalysator enthält 1,5 Gew.% Calciumoxid und 2,7 Gew.% Natriumsalze, berechnet als Natriumoxid. Die Passivierung des Katalysators wird durch Erhitzen in einem Wasserstoffstrom auf 330 °C aufgehoben.

Nach dem Abkühlen wird das Hochdruckgefäß in Rieselfahrweise bei 270 bar Wasserstoffdruck stündlich mit 0,4 l Adipodinitril, 1,2 l flüssigem Ammoniak sowie 4,5 l rohem Hydriergemisch beschickt. Hierbei hält man eine Temperatur von 140 °C ein. Die gaschromatographische Analyse des rohen Hexamethylendiamin nach Verdampfen des Ammoniaks aus dem Hydriergemisch ergibt 99,69 Gew.% Hexamethylendiamin, 0,06 Gew.% 1,2-Diaminocyclohexan und 0,12 Gew.% 2-Aminocyclopentylamin.

**Patentansprüche**

1. Geformte Katalysatormassen, enthaltend metallische Kobalt- und/oder Nickelteilchen und ein Gleitmittel, dadurch gekennzeichnet, daß die metallischen Kobalt- und/oder Nickelteilchen durch Reduktion von Kobalt- und/oder Nickeloxidteilchen mit einem Gehalt an Alkali- und/oder Erdalkalioxiden von weniger als 0,1 Gew.% bei einer Temperatur von ≤ 500 °C erhalten worden sind und die geformten Katalysatormassen eine Druckhärte von mehr als 294.10² KPa (300 kp/cm²) haben.

2. Geformte Katalysatormassen nach Anspruch 1, gekennzeichnet durch eine Druckhärte von 343.10² bis 1 470.10² KPa (350 bis 1 500 kp/cm²).

3. Geformte Katalysatormassen nach den Ansprüchen 1 und 2, dadurch gekennzeichnet, daß sie 1 bis 5 Gew.% Gleitmittel enthalten.

4. Geformte Katalysatormassen nach den Ansprüchen 1 bis 3, dadurch gekennzeichnet, daß sie Graphit enthalten.

5. Geformte Katalysatormassen nach den Ansprüchen 1 bis 4, dadurch gekennzeichnet, daß sie zusätzlich einen Gehalt an Mangan, Chrom und/oder Kupfer haben.

6. Geformte Katalysatormassen nach den Ansprüchen 1 bis 5, dadurch gekennzeichnet, daß sie zusätzlich einen Gehalt an Pyro- und/oder Polyphosphorsäure haben.

7. Verfahren zur Herstellung von geformten Katalysatormassen nach den Ansprüchen 1 bis 6 durch Reduktion von Kobalt- und/oder Nickeloxid, dadurch gekennzeichnet, daß man

a) Kobalt- und/oder Nickeloxidteilchen durch Behandeln mit Wasserstoff bei einer Temperatur von 250 bis 500 °C zu metallischen Kobalt- und/oder Nickelteilchen reduziert

b) die metallischen Kobalt- und/oder Nickelteilchen durch Behandeln mit molekularem Sauerstoff enthaltendem Inertgas passiviert

c) die passivierten Kobalt- und/oder Nickelteilchen mit Gleitmitteln zu Formkörpern verpreßt und

d) die passivierten Kobalt- und/oder Nickelteilchen sowie Gleitmittel enthaltenden Formkörper durch Behandeln mit Wasserstoff bei einer Temperatur von 250 bis 500 °C aktiviert.

8. Verfahren nach Anspruch 7, dadurch gekennzeichnet, daß man die Kobalt- und/oder Nickeloxidteilchen bis zu einem Reduktionsgrad von ≥ 95 % reduziert.

9. Verfahren nach Anspruch 7, dadurch gekennzeichnet, daß man beim Passivieren der metallischen Kobalt- und oder Nickelteilchen einen Reduktionsgrad von 80 % nicht unterschreitet.

10. Verfahren nach den Ansprüchen 7 bis 9, dadurch gekennzeichnet, daß man die Kobalt- und/oder Nickelteilchen sowie Gleitmittel enthaltende Formkörper zusätzlich mindestens einmal durch Behandeln mit molekularem Sauerstoff enthaltendem Inertgas passiviert und anschließend durch Behandeln mit Wasserstoff bei einer Temperatur von 250 bis 500 °C aktiviert.

11. Verwendung von geformten Katalysatormassen nach den Ansprüchen 1 bis 10 für Hydrierung von organischen Verbindungen.

## Claims

1. A molded catalyst material containing metallic cobalt and/or nickel particles and a lubricant, wherein the said metallic particles are obtained by reduction of cobalt oxide and/or nickel oxide particles containing less than 0.1 % by weight of alkali metal oxides and/or alkaline earth metal oxides, at ≤ 500 °C, and the molded catalyst material has an indentation hardness greater than $294 \times 10^2$ kPa (300 kp/cm²).

2. A molded catalyst material as claimed in claim 1, having an indentation hardness of from $343 \times 10^2$ to $1\,470 \times 10^2$ kPa (from 350 to 1 500 kp/cm²).

3. A molded catalyst material as claimed in claims 1 and 2, which contains from 1 to 5 % by weight of a lubricant.

4. A molded catalyst material as claimed in claims 1 to 3, which contains graphite.

5. A molded catalyst material as claimed in claims 1 to 4, which additionally contains manganese, chromium and/or copper.

6. A molded catalyst material as claimed in claims 1 to 5, which additionally contains pyrophosphoric and/or polyphosphoric acid.

7. A process for the preparation of a molded catalyst material as claimed in claims 1 to 6 by reduction of cobalt oxide and/or nickel oxide, wherein

a) cobalt oxide and/or nickel oxide particles are reduced by treatment with hydrogen at from 250 to 500 °C to give metallic cobalt and/or nickel particles,

b) the metallic cobalt and/or nickel particles are passivated by treatment with an inert gas containing molecular oxygen,

c) the passivated cobalt and/or nickel particles are pressed together with a lubricant to give moldings and,

d) the moldings containing the passivated cobalt and/or nickel particles and the lubricant are activated by treatment with hydrogen at from 250 to 500 °C.

8. A process as claimed in claim 7, wherein the cobalt oxide and/or nickel oxide particles are reduced until the degree of reduction is ≥ 95 %.

9. A process as claimed in claim 7, wherein, when the metallic cobalt and/or nickel particles are passivated, the degree of reduction does not fall below 80 %.

10. A process as claimed in claims 7 to 9, wherein the moldings containing the cobalt and/or nickel particles and the lubricant are again passivated by treatment with an inert gas containing molecular oxygen and then activated by treatment with hydrogen at from 250 to 500 °C, these treatments being carried out one or more times.

11. Use of a molded catalyst material as claimed in claims 1 to 10 for hydrogenation of organic compounds.

## Revendications

1. Catalyseurs façonnés, contenant du cobalt et(ou) du nickel en particules et un lubrifiant, caractérisés en ce que les particules de cobalt et(ou) de nickel métallique(s) ont été obtenues par réduction, à une température inférieure ou égale à 500 °C, de particules d'oxyde de cobalt et(ou) d'oxyde de nickel avec une teneur en oxydes de métaux alcalins et(ou) alcalino-terreux inférieure à 0,1 % en poids et les catalyseurs façonnés possèdent une résistance à la compression supérieure à $294.10^2$ kPa.

2. Catalyseurs façonnés suivant la revendication 1, caractérisés en ce que leur résistance à la compression est comprise entre $343.10^2$ et $1\,470.10^2$ kPa.

3. Catalyseurs façonnés suivant les revendications 1 et 2, caractérisés en ce qu'ils contiennent entre 1 et 5 % en poids de lubrifiant.

4. Catalyseurs façonnés suivant les revendications 1 à 3, caractérisés en ce qu'ils contiennent du graphite.

5. Catalyseurs façonnés suivant les revendications 1 à 4, caractérisés en ce qu'ils contiennent en outre une certaine proportion de manganèse, de chrome et(ou) de cuivre.

6. Catalyseurs façonnés suivant les revendications 1 à 5, caractérisés en ce qu'ils contiennent en plus une certaine proportion d'acide pyrophosphorique et(ou) d'acide polyphosphorique.

7. Procédé de préparation de catalyseurs façonnés suivant les revendications 1 à 6 par réduction d'oxyde de cobalt et(ou) d'oxyde de nickel, caractérisé en ce que :

a) des particules d'oxyde de cobalt et(ou) de nickel sont réduites à une température comprise entre 250 et 500 °C, à l'aide d'hydrogène, en particules de cobalt et(ou) de nickel métallique(s) ;

b) les particules de cobalt et(ou) de nickel métallique(s) sont passivées par un traitement avec un gaz inerte contenant de l'oxygène moléculaire ;

c) les particules de cobalt et(ou) de nickel passivées sont comprimées avec un lubrifiant en éléments façonnés et

d) les éléments façonnés, comprenant des particules de cobalt et(ou) de nickel passivées et un lubrifiant, sont activés par de l'hydrogène à une température comprise entre 250 et 500 °C.

8. Procédé suivant la revendication 7, caractérisé en ce que les particules d'oxyde de cobalt et(ou) de nickel sont réduites jusqu'à un degré de réduction égal ou supérieur à 95 %.

9. Procédé suivant la revendication 7, caractérisé en ce que, lors de la passivation des particules de cobalt et(ou) de nickel métallique(s), on ne réduit pas le taux de réduction à une valeur inférieure à 80 %.

10. Procédé suivant les revendications 7 à 9, caractérisé en ce que les éléments façonnés, contenant les particules de cobalt et(ou) de nickel et le lubrifiant, sont soumis à au moins une passivation supplémentaire par un gaz inerte contenant de l'oxygène moléculaire, suivie d'une activation par l'hydrogène entre 250 et 500 °C.

11. Utilisation des catalyseurs façonnés suivant les revendications 1 à 10 pour l'hydrogénation de composés organiques.